# EUROPEAN PATENT SPECIFICATION

(11) **EP 2 210 085 B1**
(45) Date of publication and mention of the grant of the patent: **30.09.2015**
(21) Application number: 08842920.4
(22) Date of filing: 25.10.2008
(51) Int. Cl.: G01N 33/49, G01N 33/86, C12Q 1/56

(54) **APPARATUS AND METHOD FOR ELECTROCHEMICAL DETECTION**
VORRICHTUNG UND VERFAHREN ZUM ELEKTROCHEMISCHEN NACHWEIS
APPAREIL ET PROCÉDÉ DE DÉPÔT ÉLECTROCHIMIQUE

(30) Priority: 26.10.2007 US 983029 P
(43) Date of publication of application: 28.07.2010
(73) Proprietor: Universal Biosensors Pty Ltd, Rowville, VIC 3178 (AU)
(72) Inventor: NEWMAN, Peter Michael, Ashburton Victoria 3147 (AU); HODGES, Alastair M., Blackburn South, Victoria 3130 (AU)
(74) Representative: Gibson, Mark
(86) International application number: PCT/IB2008/002849
(87) International publication number: WO 2009/053834

(56) References cited:
- EP-A1- 1 360 933
- WO-A1-01/59425
- WO-A2-02/48707
- US-A1- 2002 053 523
- US-A1- 2003 146 113
- US-A1- 2003 146 113
- US-A1- 2005 196 820
- US-A1- 2007 009 982
- US-A1- 2007 269 854
- US-B1- 6 352 630
- US-B1- 6 352 630
- US-B1- 6 444 115

## Description

### Field of the Invention

The present invention is directed to a sensor with opposing electrodes and test strips using this sensor. The invention can be used to measure blood or plasma coagulation in assays like prothrombin time (ET) and thrombin potential, for example, in point-of-care monitoring of anticoagulants.

### Background

Various patents discuss devices and methods of detecting electrochemical substrates. For example, Jozefonvicz et al. (US Patent No. 4,304,853) describes the use of electrochemical substrates to detect proteases, including the enzymes in a coagulation system. Unfortunately, their method is not practical for point-of-care devices. The teaching, as used herein, is useful for the finding that preconditioning of the electrode(s) is necessary to improve reproducibility because the measured currents are very low. The substrate requires DMSO to assist with solubility in aqueous solutions. It also discloses a 3-electrode system with volumes of sample and reagents that are orders of magnitude greater than that required for viable point-of-care tests.

Ludin et al. (US 6,495,336) disclose electrochemical substrates that can be used for detection of thrombin, typically in a coagulation assay. The substrate provided therein is an improvement over that of Jozefonvicz *et al.* because it is apparently water soluble. They do not disclose how to use the substrate in a point-of-care device.

Frenkel et al. (US 6,352,630) discloses use of an electrochemical thrombin substrate, specifically as disclosed by Ludin *et al.* (2002), in a device that appears to be suitable for point-of-care testing. The teaching, as used herein, provides that the substrate is to be immobilized on the working electrode via the end that is opposite the electroactive group that is cleaved off. This requirement adds complexity and limits the electrode materials,and fabrication process.

Immobilization of the substrate on the working electrode is important and/or essential when using the coplanar electrodes that Frenkel *et al.* teach. This is because the relatively large distance between the working and counter electrodes prohibits any meaningful diffusion of molecules from one electrode to the other.

Similarly, the teaching provides a Ag/AgCl reference electrode, which may also be a counter electrode, and is desired for coplanar electrode configurations because the Ag/AgCl can act as a sink or source of electrons. A potential issue of this device is that the relatively small amount of electroactive product that is released, say in the reduced form, in one embodiment, can only be detected- when it is oxidized. This may result in relatively low electrochemical currents. Furthermore, the preparation of a Ag/AgCl electrode adds to the expense of manufacturing such sensors.

Unkrig et al. (US 2003/0146113A1) also disclose the use of an electrochemical thrombin substrate that appears suitable for point-of-care devices. As used herein, the reference describes a flat 2-electrode system with a Pd working electrode and a Ag/AgCl combined reference and counter electrode, hi order to overcome the inherent low signal in this coplanar electrode design in certain embodiments the teaching regenerates the electroactive group by coupling its detection to the oxidation of glucose. An advantage of certain present embodiments is that the opposing electrodes can automatically regenerate the detected species without the need for coupling to another redox reaction.

Opalsky et al. (EP 1,234,053 B1) also describe a coagulation sensor that uses an electrochemical substrate. In certain embodiments, the teaching discloses a complex cartridge that preferably has a pump to move the sample within the cartridge to mix the sample plus reagent past coplanar electrodes. In contrast, in certain embodiments herein, the opposing electrodes permit a simple strip that does not benefit from a pump as reagent can passively diffuse throughout the smaller volume of sample.

Opposing electrode biosensors are disclosed by Hodges et al. (US 6,284,125 B1) to measure the concentration of a redox species. In particular, a redox species generated by the oxidation of glucose and from this the glucose concentration was calculated. An opposing electrode sensor can consist of:
(1) Working and counter electrodes (and optionally a reference electrode in any configuration) spaced by a predetermined distance and;
(2) The electrodes facing each other while lying on different but approximately parallel planes and;
(3) Selecting the spacing between the working electrode and the counter electrode so that reaction products from the counter electrode can diffuse to the working electrode.

Opposing electrodes are disclosed by Newman and Chatelier (PCT/IB2007/001990) for a coagulation sensor but the disclosure is limited to using magnetic particle (im)mobility to detect coagulation and makes no teaching of electrochemical substrates.

Ohara et al. (US 6,620,310 B1) describe detecting a change in viscosity of a coagulating sample by detecting the decrease in steady state current through opposing electrodes. Again the disclosure does not relate to an electrochemical substrate, instead the change in diffusion of a redox couple is detected upon coagulation.

Hodges et al. (US 6,444,115) relates to the measurement of the progress of a chemical reaction that generates an electroactive reaction product that is subsequently detected at an electrode amperometrically or coulometrically.

None of the prior art describes the use of opposing electrodes to detect an electrochemical substrate. Nor do they describe the unexpected discovery that when using opposing electrodes it is preferable not to immobilize the electrochemical substrate on the working electrode.

### Summary

Embodiments of the present invention are directed to an opposing-electrode sensor with a working electrode, a counter electrode, a reagent selected from thromboplastin, and an electrochemical thrombin substrate, wherein the thromboplastin is coated onto one of the electrodes and the electrochemical thrombin substrate is coated onto the other electrode, wherein the sensor detects cleavage of the electrochemical thrombin substrate. The electrochemical thrombin substrate can be coated on the counter electrode. These sensors can be used as part of a test strip for testing coagulation times in blood.

The invention also relates to a test strip for measuring blood or plasma coagulation time in a sample, which comprises a sensor as defined herein.

The invention also relates to a method of measuring blood or plasma coagulation comprising:
adding a blood sample from a patient to an opposing-electrode sensor comprising a working electrode, a counter electrode, thromboplastin, and an electrochemical thrombin substrate, characterized in that the thromboplastin is coated onto one of the electrodes and the electrochemical thrombin substrate is coated onto the other electrode, and that the sensor detects cleavage of the electrochemical thrombin substrate; and
analyzing the sample using the sensor to determine the level of cleavage to determine at least one blood or plasma coagulation property of the sample.

Other embodiments of the invention are directed to methods of using the sensors and strips of the present invention. These methods can be practiced at the point of care.

### Brief Description of the Figures

FIG. 1 illustrates in exploded view an exemplary electrochemical strip in accordance with the present embodiments, including a bottom electrode (1), an insulating separator (2), a top electrode (3), a cover over a fill channel (4), a fill channel (5) and a reaction chamber (6).
FIG. 2 illustrates an exemplary assembled electrochemical strip in accordance with the present embodiments, including a bottom electrode (1), an insulating separator (2), a top electrode (3), a cover over a fill channel (4), a fill channel (5) and a reaction chamber (6).

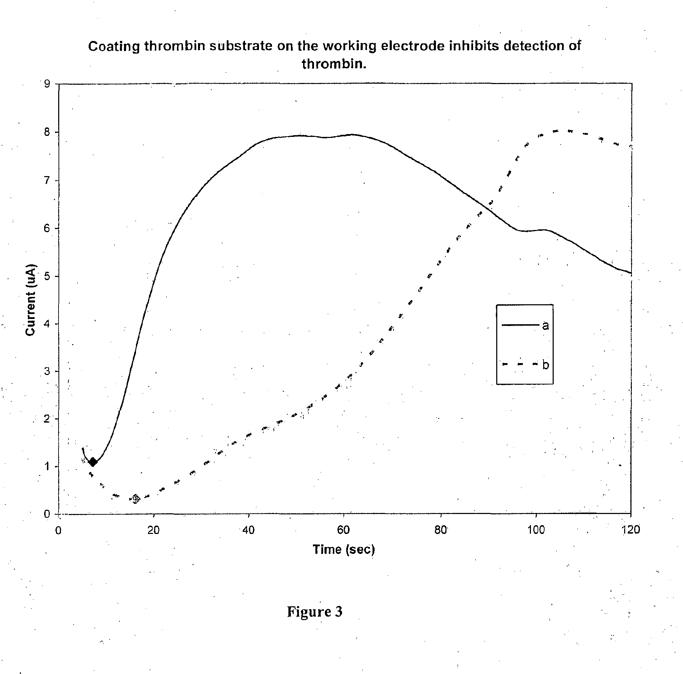

FIG. 3 illustrates an exemplary graph in accordance with the present embodiments; the figure demonstrates improved clot detection when thrombin substrate is coated on the counter electrode; time in seconds is provided as abscissa, and current in microamps is provided as ordinate; the graph labeled "a" represents thrombin substrate coated on counter electrode (the cathode in this example) and thromboplastin coated on working electrode (the anode in this example); the graph labeled "b" represents thrombin substrate coated on working electrode (anode) and thromboplastin coated on counter electrode (cathode).

### Detailed Description

Exemplary aspects of the disclosure are discussed in detail below. References to "one aspect," "an aspect," "example aspect," "various aspects," etc., may indicate that the aspect(s) of the disclosure so described may include a particular feature, structure, or characteristic, but not every aspect necessarily includes the particular feature, structure, or characteristic. Further, repeated use of the phrase "in one aspect," or "in an exemplary aspect," do not necessarily refer to the same aspect, although they may.

The present invention is directed to a sensor having opposing electrodes, i.e., a working and a counter electrode, thromboplastin, and an electrochemical thrombin substrate. In some embodiments, the electrochemical thrombin substrate is on the counter electrode. As will be described to follow, these sensors can be used by adding a sample to the sensor (optionally on a test strip) where a protease cleaves an electroactive species off the substrate. This electroactive species can be detected by redox reactions at the electrodes.

The disclosed sensor can be used in a wide variety of technologies, for example, in methods of measuring blood or plasma coagulation using assays like prothrombin time (PT) and thrombin potential, particularly in point-of-care monitoring of oral anticoagulants (e.g. coumadins like warfarin and phenprocoumon).

As discussed more below, one advantage of the present disclosure is that the opposing electrodes of certain embodiments do not need the complexity of a true reference electrode such as a Ag/AgCl electrode. Instead, in these aspects, the close proximity of the electrodes permits cycling of an electroactive species. That is, a molecule that has been reduced at the cathode can be reoxidised at the anode, resulting in a higher current. This can make it is possible to use two similar electrodes such as those formed from an inert conducting material such as carbon, Au, Pd, Pt Ir, indium oxide, mixed indium/tin oxide, and/or tin oxide, etc.

The devices and test strips of the present disclosure have an electrochemical thrombin substrate which can be dried on the counter electrode in some aspects. The electrochemical thrombin substrate can be cleaved by an enzyme, for example, a protease. Proteases are a class of enzymes that cleave peptide bonds. They have an essential role in many biological processes. For example, they are involved with food digestion, HIV replication, the blood coagulation cascade and the complement pathway.

Some proteases are specific for a particular peptide sequence while others are less selective. In either case, it can be highly desirable to detect and/or quantify protease activity as this provides an insight into the biological process. As one of skill in the art will appreciate, any naturally occurring or synthetically engineered protease or other cleaving enzyme can be detected or otherwise used in the present invention provided the enzyme is able to recognize and cleave the desired bond or bonds in the electrochemical thrombin substrate being used.

A number of useful peptide substrates are commercially available for a range of proteases. Typically a protease cleaves a dye or fluorescent group from these substrates, resulting in a change in color or fluorescence. It is also possible to create substrates with electroactive groups that can be detected electrochemically after cleavage. The amino acid sequence of a peptide substrate determines specificity for a protease. Thus, in some aspects, the specificity of a protease detection method can be changed by selection of a substrate specific for the protease of interest.

One exemplary protease detection system that aspects of the present disclosure can detect is the blood .coagulation cascade. The blood coagulation cascade consists of a series of protease reactions and serves as a model system, with practical applications, to investigate the detection of proteases. The time taken for a sample of blood or plasma to coagulate can be indicative of various disease states and useful for monitoring anticoagulation therapy. The specificity of such clotting tests usually comes, not from how the coagulation is detected, but from the nature of the reagent(s) used to initiate coagulation. Thus, the present aspects are applicable to a wide range of clotting tests.

For example, one area of use for the present disclosure is methods of monitoring anticoagulation by oral vitamin K antagonists such as warfarin and phenprocoumon at the point-of-care. These drags are generally monitored using a prothrombin time test and results are reported as an international normalized ratio (INR). There is a trend towards performing these tests using point-of-care devices that provide a result within a couple of minutes using blood derived from a simple finger-prick. This approach is usually more convenient than traditional venipuncture and plasma preparation but such devices require a method for detecting coagulation in whole blood. The present aspects improve on techniques that address this issue.

Plasma coagulation can be detected in a number of ways. For example, the gelling of the sample can be detected by increased resistance to particle (typically magnetic) movement through the sample or by a change in fluidity of the sample. The gel point may also correspond to increasing turbidity of plasma, which can be detected optically. Electrochemical methods can be used to detect changes in the viscosity of a sample by measuring changes in impedance, for example, as found in US Patent Nos. 6,673,622; 6,066,504; and 6,060,323; 6,046,051.

Coagulation can be indirectly detected using artificial peptide substrates for thrombin in accordance with the aspects. For example, chromogenic substrates can be cleaved by thrombin (or other coagulation enzymes) and a dye group may be released. This produces a detectable color change in the solution. Other substrates can be provided where the cleaved group is electrochemically active (can be oxidized and/or reduced at an electrode) and can be detected in an electrochemical sensor. This type of substrate is referred to as an electrochemical substrate and is the subject of certain aspects herein.

For the purposes of the present aspects an electrochemical substrate is defined, as a chemical compound which is cleaved by an enzyme to release an electroactive leaving group. The intact substrate has little, or different, electrochemical activity, under the detection conditions, compared to the free leaving group that is released by cleavage.

An electroactive species can be detected amperometrically in embodiments of the present invention by, for example, using either a two or three electrode system. A three electrode system can have a working, a counter, and a reference electrode. The reference electrode measures the voltage of the solution near the surface of the working electrode. The potential between the working and counter electrode can be adjusted so that the voltage between the working and reference electrode is set to the desired value. The magnitude of the current through the circuit connecting the working and counter electrode is determined by the rate of reduction or oxidation at the solution interface of the working electrode. Thus, it is at the working electrode where the reaction of interest takes place. The system is designed so that reaction at the working electrode, and not the counter electrode, limits the current.

In an aspect, a two electrode system does not have a separate reference electrode. Instead the voltage between the conductors of the two remaining electrodes can be set. The working electrode can again be defined as the electrode at which the reaction of interest takes place. This will be the electrode at which the current-limiting reaction occurs.

The inventors have surprisingly found that electrochemical sensors having opposing electrodes can have a number of advantages over those using co-planar electrodes, including, but not limited to the following:
(1) Small electroactive species can easily diffuse from one opposing electrode to the other. This can result in a recycling of the reduced and oxidized forms, effectively amplifying the signal. This is not possible in co-planar electrodes as the distances are too great for species to diffuse the required distance to the working electrode in the time available.
(2) As one of skill in the art will appreciate, any redox reaction must have an oxidation and a reduction component. In opposing electrode sensors the electroactive species of interest can be oxidized at one electrode and reduced at the other in the aforementioned recycling. In coplanar electrode sensors, extra effort is required to ensure that the reaction at the counter electrode can occur. For example the counter electrode may be Ag/AgCl, or a large voltage may be applied, or a complementary electroactive species may be required.
(3) The close proximity of opposing electrodes to each other means that most of the potential difference between the electrodes is accounted for by the voltage across the electrode interfaces and not across the bulk of the solution. In contrast, co-planar electrodes may have a considerable IxR voltage drop across the sample, particularly if the solution between the electrodes has a low ionic strength in an embodiment.
(4) Manufacture of opposing electrodes can be easier than co-planar because to form coplanar electrodes it can be necessary to either deposit or ablate metal (or another conductive material) to create distinct electrodes that are electrically isolated from each other. In contrast, it can be advantageous to have a conductive surface covering the entire material that forms each opposing electrode. This means, for example, that a simple process like sputter coating can be used to coat the electrodes and specific patterns in the conductor are not required. The opposing electrode area that contacts the sample can be defined by the insulating spacer that separates the electrodes.
(5) Using opposing electrodes can avoid the requirement in certain embodiments of Frenkel et al. (US 6,352,630; 2002) requiring immobilization of an electrochemical substrate in a specific orientation. In opposing electrode sensors the electrochemical substrate can be free to dissolve in the sample and its orientation is irrelevant. This simplifies the manufacturing process.

One or more aspects are related to a device, in the configuration of a strip, which measures protease activities. For example, to monitor blood coagulation. The strip connects electrically to a meter that applies a predetermined voltage or current to the strip and measures the resulting current or voltage. As one of skill in the art will appreciate, these methods are known, for example, as amperornetry or galvanostatic electrochemical techniques respectively. The meter can also thermally regulate the strip, calculate, record, display and/or transmit the result of the reaction.

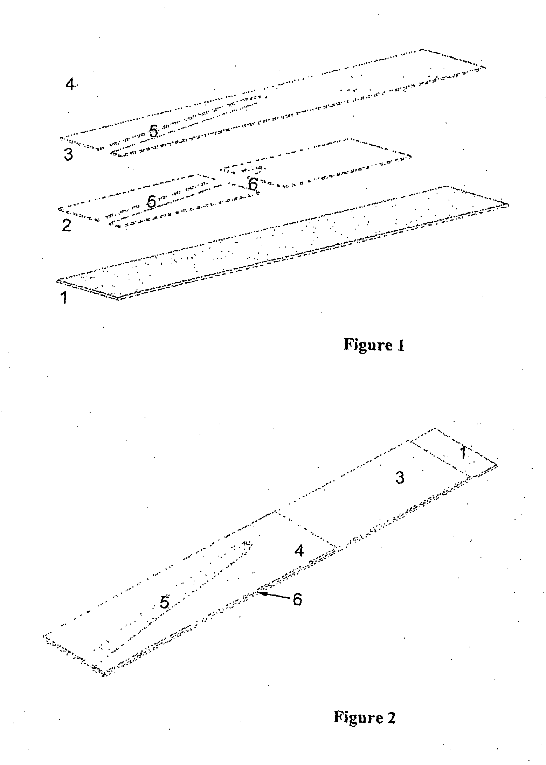

An exemplary strip, shown in FIGS. 1 and 2, can comprise two electrodes, 1 and 3, in an opposing configuration. The electrodes can be electrically insulated from each other, by a plastic separator, 2, or its equivalent. In some aspects the electrodes can be less than 0.5mm apart and in certain embodiments between 0.05 and 0.15mm apart.

The separator, 2, can have a gap in it that forms a cavity between the electrodes and defines a reaction chamber, 6. The electrode surfaces are electrically conductive and in electrical contact with the meter.

In certain aspects, the surfaces are sputter coated onto a plastic support. Suitable coatings may be known to those skilled in the art and may include platinum, indium, carbon, mixed indium/tin oxide, and tin oxide but preferably palladium or gold.

The surfaces on each of the electrodes can be the same or different. The conductive surfaces face each other. A fill channel, 5, can allow a sample to be introduced into the reaction chamber while the reaction chamber can be maintained at a set temperature within the meter.

In certain aspects the electrochemical thrombin substrate is not coated onto the working electrode. After addition of the sample (e.g., blood), cleavage of the thrombin substrate releases an electroactive species and a detectable change in the electrochemical activity take place.

In certain aspects, the disclosed strip consists of two electrodes in an opposing configuration. The electrodes can comprise palladium sputter coated onto a plastic support. An insulating separator layer, 0.1 mm thick, can define the separation of the electrodes. One or more gaps in the separator can define reaction chamber(s). A fill channel can allow sample to be conducted into the reaction chamber(s) by capillary action.

The reagent contains thromboplastin, necessary for a prothombin time test.

The reagent contains dilute thromboplastin, necessary for a thrombin generation test from which parameters like lag-time, thrombin peak and endogenous thrombin potential can be derived.

In some aspects it is advantageous to coat one or more electrodes with an electroactive compound that ensures the counter electrode does not limit the current. For example, if the cathode is the counter electrode then coating it with iron (III) EDTA provides a reducible compound to support the detection (in this embodiment, by oxidation) of the species of interest. This can be particularly advantageous if the species of interest is not completely electrochemically reversible.

It has been found to be advantageous to dry thromboplastin and the electrochemical thrombin substrate on opposite electrodes to prevent adverse reactions between the two reagents. In one exemplary embodiment, the electrochemical thrombin substrate (Toluolsulfonyl-glycyl-prolinyl-arginin-4-amido-2-chlorophenol) can be coated onto the counter electrode and thromboplastin can be coated onto the working electrode. In this example, the leaving group of the thrombin substrate is cleaved in the reduced form. Here, it is the species that is being detected and limits the current. Since the leaving group is in the reduced form it can be oxidized on the anode to pass current, and thus the anode is the working electrode.

In general, it would be expected that coating the thrombin substrate on the working electrode would produce faster clot detection because the leaving group is released at the electrode where it is detected. However, in some embodiments, the opposite is true. For example, FIG. 3 shows that the reaction kinetics was faster when the thrombin substrate was dried on the counter electrode (cathode) rather than the working electrode (anode). The faster reaction rate of these embodiments can make determination of the clot time more consistent.

Many biological assays (e.g., thrombin generation assays) involve calculating reaction rates. In certain embodiments, it is observed that it can be easier to measure the reaction kinetics of a chemically generated electroactive species when the species is diffusing to the working electrode rather than being generated at the working electrode. See, e.g., US Patent Nos. 5,942,102 and 6,444,115. This is another reason why in certain embodiments it can be preferable to coat electrochemical thrombin substrates away from the working electrode.

In another aspect, the electrochemical thrombin substrate and thromboplastin are formulated so as not to adversely interact, which can allow all or the reagent components to be coated together on the counter electrode. This has an advantage of leaving the working electrode free of any reagent that can foul it. Fouling is less of a concern on the counter electrode because that electrode is not limiting the current. Fouling of the working electrode may not be immediately apparent and often only becomes apparent after storage where it can be a cause of shortened shelf life for a product embodying the present invention. Thus, coating on the counter electrode can avoid the risk of fouling the working electrode.

The test strips of the present disclosure can also include means for verifying strip and/or sample integrity. In one aspect, the strip has a reaction chamber containing reagents that function to verify strip and/or sample integrity. The reagents can include coagulation factors that are otherwise deficient in the sample. This reaction chamber can produce a coagulation time that is approximately normal, regardless of the degree of deficiency in the sample, and be used to demonstrate strip integrity when the coagulation time falls within predetermined limits.

In another aspects strip integrity may be verified by including an electroactive compound in the reagent. Such a compound may be different from the electrochemical thrombin substrate, which may perhaps only release an electroactive group on enzymatic cleavage. The compound that monitors strip integrity may change (increase or decrease) electrochemical activity under storage conditions (e.g. elevated temperature or humidity) that may damage other components of the strip. Examples of such compounds may include 4-amino-2-chlorophenol, ferricyanide, ferrocyanide, and the organic N-oxide or nitroso compounds disclosed in U.S. Patent Application No. US 2005/0123441. The electrochemical activity of such compounds can be compared to predetermined limits for the batch of strips. If the value falls outside the limits then the meter may give an error message instead of a result.

Reagents to monitor strip integrity may be in the same reaction chamber that detects substrate cleavage or they may be in different chambers. In the aspect where chambers are separate then the chambers may be electrically isolated by discontinuity of the electrodes or, in an alternative aspect the chambers may use electrically connected electrodes. Where the chambers share a continuous electrode, the signal detected by the meter may be a combination of the chambers. Interference between the integrity and cleavage reaction signals can be minimized by detecting the integrity reaction before or after the cleavage reaction and/or by reducing the cross-reacting electrode area.

Some aspect of the present are directed to an on-board control suitable for monitoring the viability of electrochemical biosensor strips, such as, but not limited to those disclosed herein. Electrode materials, and the coating on them, are chosen such that a potential difference is established between the electrodes. This voltage can be measured directly or the current that it induces is measured. In some aspects, the sensor briefly behaves like a battery, generating charge. The potential difference breaks down after a relatively short time and does not interfere with the subsequent test reaction.

Such controls are useful in, for example, point-of-care tests, based on a meter that measures an electrochemical reaction in a. disposable strip, which are becoming more common. There is a need to ensure that test results are accurate so controls to ensure strip integrity are desirable. For end users, e.g., a patient, the most convenient control is one that is "on-board" the strip and verifies the integrity of the same strip that is being tested. Examples of on-board controls can be found in the area of point-of-care coagulation devices already on the market (e.g., Coaguchek XS, INRatio). Also, US2005123441 A1 provides a concise introduction to the advantages of an on-board control.

Electrochemical biosensors typically become more complicated the more isolated the control reaction is from the test reaction. For example, in order of increasing complexity:
a. A strip with a single reaction chamber that includes both control reaction and test reaction is comparatively easy to manufacture.
b. A strip with control and test chambers that are separate but electrically connected is more complicated as it requires deposition of multiple reagents in different areas.
c. A strip with control and test chambers that are not only separate but also electrically isolated can be quite challenging to manufacture economically at scale. It usually requires deposition or ablating electrode material in a pattern.

However, the difficulty in finding suitable control reaction chemistry is in the reverse order to that above.
a. A strip with separate, and electrically isolated, control and test chambers will not have any problems with one reaction (e.g. control) interfering with the other (e.g. test).
b. A strip with separate, but electrically connected, control and test chambers may suffer from electrical interference between the chambers as electrons from on reaction are indistinguishable from electrons from the other but the control and test will not interfere with each other chemically.
c. A strip with a single reaction chamber requires components that will not interfere with each other chemically or cause interfering electrical signals.

Further disclosed is a novel way of assessing a control reaction in an electrochemical sensor as well as a way to prevent electrical signals from the control reaction and test reaction from interfering with each other.

US200512344 A1 discloses an on-board control reaction that utilizes N-oxide or nitroso compounds that become reduced upon exposure to conditions that may damage strip performance. The change in on-board control can be detected optically or electrochemically. The electrochemical detection method disclosed in US2005123441 A1 involves applying -700mV, relative to Ag/AgCl, across the working electrode and then applying -100mV.

The present disclosure can be advantageous because the inventive controls only require 1-2 sec instead of 4.5sec for some previous devices. Our control does not risk applied voltage interfering with the test.

Disclosed is an on-board control for monitoring the viability of electrochemical sensors that detect blood coagulation. The control reaction reagent, which measures strip viability, is contained within the same chamber as the test reaction reagents, which measure clot time. The signal from the control reaction is differentiated from that of the test by the time at which they occur. Specifically, the control reaction is assessed in the first few seconds after adding the sample and then the test reaction is assessed.

The control reaction can be created by coating, on one electrode, reagent that contains a low (e.g. 0.5mM) concentration of ferricyanide and on the other electrode reagent without ferricyanide. The difference in electrode chemistry creates a voltage across the electrodes when the sample is added. This voltage can be measured directly with a voltmeter or the current through in an external circuit can be recorded by an ammeter.

The electrochemical voltage can dissipate by at least two methods: Current flowing through an external circuit effectively flattens the electrochemical battery and diffusion of ferricyanide from one electrode to the other decreases the ferricyanide concentration gradient and thus the voltage. Once the signal from the control reaction has dissipated then the analysis of the test reaction can begin. This may, for example, consist of applying 0.3-0.4V across the electrodes and measuring the resulting amperommetric current.

The concentration of ferricyanide is sufficiently low so that it does not substantially interfere with the detection of the test reaction. As one of skill in the art will appreciate, however, using alternatives to ferricyanide can be produced. For example, iodine, ascorbate, ferrocyanide, 4-amino-2-chlorophenol can produce a battery effect.

The battery effect is measured by measuring the current without any external voltage being applied across the sensor electrodes. However, it is possible to measure the current with a small voltage applied provided that the voltage does not cause substantial interference with the test reaction.

The sensors can contain two chambers. One chamber can contain the test-reaction reagents and the other the control-reaction reagents. This approach is advantageous if the components of one reaction (e.g. the control reaction) may chemically inhibit the other (e.g. the test reaction). The chambers do not need to be electrically isolated provided the test and control reaction do not interfere with each other electrically. This aspect is particularly advantageous if a neutralising agent is included in the control reaction chamber (e.g. on an opposing electrode).

Alternatively, concentrations of control-reaction reagent that would normally interfere with the test-reaction can be used. In this aspect the control chamber contains separate areas of control reagent and a neutralising agent. When the sample is added the control reagent produces a battery effect or some other electrochemical signal but is then quickly neutralised by the neutralising agent. The neutralising effect can be by a chemical reaction (e.g. iodine and ascorbate neutralise each other) or more of a physical effect such as precipitation (e.g. Co²⁺ or Mn²⁺ ions precipitate ferricyanide). Precipitation of an electroactive compound can render it incapable of interacting with an electrode.

In an aspect that measures blood or plasma coagulation, a meter can maintain the sensor at 37°C. The electrical signal detected by the meter can be interpreted to calculate the clot time. For example, the clot time can be defined as the point where the signal, or the rate of change thereof, reaches a threshold. The clot time can be reported, displayed, or otherwise outputted with the units of time or it can be converted into different units like INR by using calibration data assigned to the sensor and/or meter.

Other results may also be calculated and reported. For example, with the right reagents, the area under the curve can indicate the endogenous thrombin potential, which can similarly be reported. The assay result may be reported by displaying on the meter's screen but it can also be stored in memory and/or transmitted to another device.

Also disclosed are kits having a sensor or test strip described herein. Such kits may be sterile packaged. The test strips or sensors can come in individual packages as well as multipacks. Optionally, the kits include a meter for interacting with the test strips or sensor and/or instructions for using the kit, test strip, or sensor.

## Claims

1. An opposing-electrode sensor comprising a working electrode, a counter electrode, thromboplastin, and an electrochemical thrombin substrate, wherein the thromboplastin is coated onto one of the electrodes and the electrochemical thrombin substrate is coated onto the other electrode, wherein the sensor detects cleavage of the electrochemical thrombin substrate.

2. The sensor of claim 1, wherein the electrochemical thrombin substrate is not immobilized on the working electrode.

3. The sensor of claim 1 or claim 2, **characterized in that** the electrochemical thrombin substrate comprises a peptide bound to a leaving group, wherein the leaving group becomes electroactive once cleaved by thrombin.

4. The sensor of any one of claims 1-3, further comprising a meter connected to the sensor, wherein the meter determines a coagulation time for a sample.

5. The sensor of claim 4, wherein the meter displays or outputs a result comprising the coagulation time or a result or value dependent upon the coagulation time.

6. The sensor of any one of the preceding claims, further comprising a meter connected to the sensor, wherein the meter displays or outputs a result comprising a parameter of thrombin generation in the sample.

7. A test strip for measuring blood or plasma coagulation time in a sample, which comprises a sensor of any one of claims 1-6.

8. The test strip of claim 7, which further comprises a means for verifying strip integrity.

9. A method of measuring blood or plasma coagulation comprising:
adding a blood sample from a patient to an opposing-electrode sensor comprising a working electrode, a counter electrode, thromboplastin, and an electrochemical thrombin substrate, **characterized in that** the thromboplastin is coated onto one of the electrodes and the electrochemical thrombin substrate is coated onto the other electrode, and that the sensor detects cleavage of the electrochemical thrombin substrate; and
analyzing the sample using the sensor to determine the level of cleavage to determine at least one blood or plasma coagulation property of the sample.

10. The method of claim 9, wherein the method is performed at the point of care.

11. The method of claim 9 or claim 10, wherein the method further comprises a meter connected to the sensor, wherein the meter displays or outputs a result comprising a parameter of thrombin generation in the sample.

12. The method of any one of claims 9-11, wherein the patient is taking an anticoagulant medication.

13. The method of claim 12, wherein the anticoagulant medication is a vitamin K antagonist.

14. The method of any one of claims 9-13, wherein the sample is from the patient by a finger prick of the patient.

## Patentansprüche

1. Sensor mit gegenüberliegenden Elektroden, der eine Arbeitselektrode, eine Gegenelektrode, Thromboplastin und ein elektrochemisches Thrombinsubstrat umfasst, wobei das Thromboplastin auf eine der Elektroden aufgetragen ist und das elektrochemische Thrombinsubstrat auf die andere Elektrode aufgetragen ist, wobei der Sensor eine Spaltung des elektrochemischen Thrombinsubstrats detektiert.

2. Sensor nach Anspruch 1, wobei das elektrochemische Thrombinsubstrat nicht auf der Arbeitselektrode immobilisiert ist.

3. Sensor nach Anspruch 1 oder Anspruch 2, **dadurch gekennzeichnet, dass** das elektrochemische Thrombinsubstrat ein an eine Austrittsgruppe gebundenes Peptid beinhaltet, wobei die Austrittsgruppe nach dem Spalten durch Thrombin elektroaktiv wird.

4. Sensor nach einem der Ansprüche 1-3, der ferner ein mit dem Sensor verbundenes Messgerät umfasst, wobei das Messgerät eine Gerinnungszeit für eine Probe ermittelt.

5. Sensor nach Anspruch 4, wobei das Messgerät ein Ergebnis anzeigt oder ausgibt, das die Gerinnungszeit oder ein Ergebnis oder einen Wert beinhaltet, das/der von der Gerinnungszeit abhängig ist.

6. Sensor nach einem der vorherigen Ansprüche, der ferner ein mit dem Sensor verbundenes Messgerät umfasst, wobei das Messgerät ein Ergebnis anzeigt oder ausgibt, das einen Parameter der Thrombinbildung in der Probe beinhaltet.

7. Teststreifen zum Messen der Blut- oder Plasmagerinnungszeit in einer Probe, der einen Sensor nach einem der Ansprüche 1-6 beinhaltet.

8. Teststreifen nach Anspruch 7, der ferner ein Mittel zum Verifizieren der Streifenintegrität beinhaltet.

9. Verfahren zum Messen der Blut- oder Plasmagerinnung, das Folgendes beinhaltet:
Geben einer Blutprobe von einem Patienten zu einem Sensor mit gegenüberliegenden Elektroden, der eine Arbeitselektrode, eine Gegenelektrode, Thromboplastin und ein elektrochemisches Thrombinsubstrat umfasst, **dadurch gekennzeichnet, dass** das Thromboplastin auf eine der Elektroden aufgetragen ist und das elektrochemische Thrombinsubstrat auf die andere Elektrode aufgetragen ist und dass der Sensor eine Spaltung des elektrochemischen Thrombinsubstrats detektiert; und
Analysieren der Probe mit dem Sensor, um den Grad an Spaltung zu bestimmen, um wenigstens eine Blut- oder Plasmagerinnungseigenschaft der Probe zu bestimmen.

10. Verfahren nach Anspruch 9, wobei das Verfahren patientennah durchgeführt wird.

11. Verfahren nach Anspruch 9 oder Anspruch 10, wobei das Verfahren ferner ein mit dem Sensor verbundenes Messgerät beinhaltet, wobei das Messgerät ein Ergebnis anzeigt oder ausgibt, das einen Parameter der Thrombinbildung in der Probe beinhaltet.

12. Verfahren nach einem der Ansprüche 9-11, wobei der Patient ein gerinnungshemmendes Medikament einnimmt.

13. Verfahren nach Anspruch 12, wobei das gerinnungshemmende Medikament ein Vitamin-K-Antagonist ist.

14. Verfahren nach einem der Ansprüche 9-13, wobei die Probe vom Patienten durch einen Fingerstich des Patienten genommen wird.

## Revendications

1. Capteur à électrodes en opposition comprenant une électrode de travail, une contre-électrode, une thromboplastine et un substrat électrochimique de la thrombine, dans lequel la thromboplastine est enduite sur l'une des électrodes et le substrat électrochimique de la thrombine est enduit sur l'autre électrode, le capteur détectant le clivage du substrat électrochimique de la thrombine.

2. Capteur de la revendication 1, dans lequel le substrat électrochimique de la thrombine n'est pas immobilisé sur l'électrode de travail.

3. Capteur de la revendication 1 ou de la revendication 2, **caractérisé en ce que** le substrat électrochimique de la thrombine comprend un peptide lié à un groupement partant, le groupement partant devenant électroactif une fois clivé par la thrombine.

4. Capteur de l'une quelconque des revendications 1-3 qui comprend en outre un compteur connecté au capteur, le compteur déterminant le temps de coagulation d'un échantillon.

5. Capteur de la revendication 4, dans lequel le compteur affiche ou délivre un résultat comprenant le temps de coagulation ou un résultat ou une valeur qui dépend du temps de coagulation.

6. Capteur de l'une quelconque des revendications précédentes qui comprend en outre un compteur connecté au capteur, le compteur affichant ou délivrant un résultat incluant un paramètre indicateur de la formation de thrombine dans l'échantillon.

7. Bandelette réactive permettant de mesurer le temps de coagulation sanguine ou plasmatique dans un échantillon, qui comprend un capteur de l'une quelconque des revendications 1-6.

8. Bandelette réactive de la revendication 7, qui comprend en outre un moyen de vérifier l'intégrité de la bandelette.

9. Méthode de mesure de la coagulation sanguine ou plasmatique qui comprend :
l'addition d'un échantillon de sang prélevé chez un patient sur un capteur à électrodes en opposition comprenant une électrode de travail, une contre-électrode, une thromboplastine et un substrat électrochimique de la thrombine, **caractérisé en ce que** la thromboplastine est enduite sur l'une des électrodes et le substrat électrochimique de la thrombine est enduit sur l'autre électrode, et **en ce que** le capteur détecte le clivage du substrat électrochimique de la thrombine ; et
l'analyse de l'échantillon au moyen du capteur pour mesurer le degré de clivage et déterminer ainsi au moins une propriété de la coagulation sanguine ou plasmatique de l'échantillon.

10. Méthode de la revendication 9, qui est effectuée au lieu d'intervention.

11. Méthode de la revendication 9 ou de la revendication 10 qui comprend en outre un compteur connecté au capteur, le compteur affichant ou délivrant un résultat incluant un paramètre indicateur de la formation de thrombine dans l'échantillon.

12. Méthode de l'une quelconque des revendications 9-11, dans laquelle le patient prend un médicament anticoagulant.

13. Méthode de revendication 12, dans laquelle le médicament anticoagulant est un antagoniste de la vitamine K.

14. Méthode de l'une quelconque des revendications 9-13, dans laquelle l'échantillon est obtenu par piqûre au doigt du patient.
